# EUROPEAN PATENT APPLICATION

(11) **EP 1 785 420 A1**
(43) Date of publication of application: **16.05.2007**
(21) Application number: 05024847.5
(22) Date of filing: 14.11.2005
(51) Int. Cl.: C07D 417/12, C07D 417/14, A61K 31/505, A61P 35/00

(54) **Thiazole analogues and uses thereof**

(71) Applicant: 4SC AG, 82152 Martinsried (DE)
(72) Inventor: Ehlert, Jan, Dr., 79238 Ehrenkirchen (DE); Herz, Thomas, Dr., 82131 Stockdorf (DE); Krauss, Rolf, Dr., 82152 Planegg-Martinsried (DE); Kubbutat, Michael, Dr., 79227 Schallstadt (DE); Lang, Martin, Dr., 82166 Gräfelfing (DE); Pegoraro, Stefano, Dr., 82152 Planegg-Martinsried (DE); Schächtele, Christoph, Dr., 79108 Freiburg (DE); Totzke, Frank, Dr., 79098 Freiburg (DE); Zirrgiebel, Ute, Dr., 79194 Gundelfingen (DE)
(74) Representative: Vossius & Partner

(57) **Abstract**

The present invention relates to compounds of the general formula (I) and salts and physiologically functional derivatives thereof, 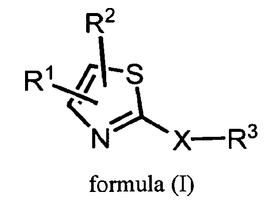 R²
is attached at the 4- or 5-position of the thiazole ring and is hydrogen, alkyl, halogen, cyano, alkoxy, haloalkyloxy, or alkylamino;
X
independently represents a divalent linkage group selected from S, O, NR⁴, SO, or SO₂;
R⁴
is hydrogen, alkyl, cycloalkyl, cycloalkylalkyl, or heterocyclyl;

is attached at the 4- or 5-position of the thiazole ring and independently represents one of the following groups of the general formula (II): 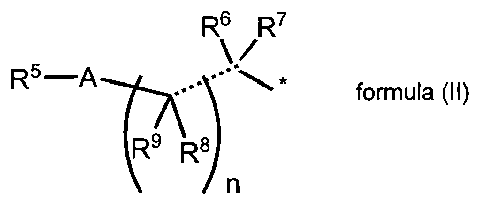 wherein
the dotted line represents a single or double bond;
*
indicates the point of attachment to the thiazole ring;
n
is 1,2, or 3.

## Description

The present invention relates to thiazoles of the general formula (I) or a salt or a physiologically functional derivative or a stereoisomer thereof, for the use as a medicament. The compounds of the invention are useful for the treatment of diseases associated with abnormal and hyperproliferation of cells in a mammal, especially humans. In particular, they are useful for the treatment of all forms of cancer.

Furthermore a process of preparing said thiazole derivatives is disclosed.

### Background of the invention

Protein kinases play a central role in the regulation of cellular functions. These include processes like cell growth and division, cell differentiation and cell death, but also many other cellular activities. Protein kinases catalyze the transfer of phosphate residues from ATP on target proteins which as a consequence of this protein kinase mediated phosphorylation change their three-dimensional structure and thereby their physiological function. Depending on the amino acid which is phosphorylated by a protein kinase these enzymes are grouped in two families, the so-called serine/threonine protein kinases and the tyrosine protein kinases.

Based on the human genome project it is known that in human beings there exist 518 DNA sequences which encode for a protein kinase-like protein sequence. For several of these 518 proteins it could be shown in the last about 20 years that modifications in their related gene sequences (e.g. point mutations, deletions or gene amplifications) result in pathological changes of the cellular activities of the corresponding protein kinase. This is in particular true for protein kinases which are involved in cell proliferation and cell cycle control, in survival of cells and cell death, in tumor angiogenesis, and in formation of tumor metastases.

Several so-called oncogenes are pathologically modified genes which in their proto-oncogenic form encode for protein kinases involved in normal, physiological regulation of cell growth and division.

Since protein kinases are key regulators of cell functions and since they can show dysregulated enzymatic activity in cells they are promising targets for the development of therapeutic agents. There are many ongoing drug discovery projects in the pharmaceutical industry with the goal to identify modulators of protein kinases. The major focus is currently on protein kinases involved in inflammation and cancer, but besides this protein kinases are currently discussed as promising targets in almost every area of diseases.

In the field of tumors the first protein kinase inhibitors (Gleevec, Iressa, Tarceva) have already reached the market. In addition, a great number of protein kinase inhibitors are currently in various phases of clinical development. In most cases these compounds are either targeting subtypes of the EGF (Epidermal Growth Factor) receptor family or of the VEGF (Vascular Endothelial Growth Factor) receptor family. All these compounds have been developed with the goal to specifically inhibit one particular protein kinase, for which there is evidence that it interferes with one of the four major molecular processes of tumor progression. These four processes are (1) cell proliferation/cell cycle control, (2) regulation of programmed cell death (apoptosis) and cell survival, (3) tumor angiogenesis and (4) tumor metastasis.

The present invention relates to thiazole derivatives which may be useful for inhibition of protein kinases involved in diseases besides cancer, but which are especially useful as antitumor agents. This includes monospecific protein kinase inhibitors, which preferentially inhibit one protein kinase which is causally involved in tumor progression, but also so-called multi-target protein kinase inhibitors, which inhibit at least two different protein kinases which either relate to the same or to two or more different molecular mechanisms of tumor progression. As an example, such a compound could be an inhibitor of tumor angiogenesis and, in addition, also a stimulator of apoptosis.

The concept of multi-target protein kinase inhibitors is a new approach although the idea of developing "multiplex protein kinase inhibitors" has already been described by J. Adams et al., Current Opinion in Chemical Biology 6, 486-492, 2002. Therein compounds are described, which, at the same time, inhibit several protein kinases, which however all are involved in one molecular mechanism of tumor progression, namely tumor angiogenesis.

The object of the present invention is solved by the subject-matter of the independent claims. Further advantageous features, aspects and details of the invention are evident from the dependent claims, the description, the figures, and the examples of the present application.

Considering the lack of currently available treatment options for the majority of the conditions associated with protein kinases like ABL1, ACV-R1, AKT1, AKT2, AKT3, ARK5, Aurora-A, Aurora-B, Aurora-C, B-RAF, BRK, CDC42BPB, CDK1, CDK2, CDK3, CDK4, CDK5, CDK6, CDK7, CDK9, CHK1, CK2, COT, CSK, DAPK1, EGF-R, EPHA1, EPHA2, EPHA4, EPHB1, EPHB2, EPHB3, EPHB4, ERBB2, ERBB4, FAK, FGF-R1, FGF-R3, FGF-R4, FGR, FLT3, GSK3-beta, HCK, IGF1-R, IKK-beta, IKK-epsilon, INS-R, IRAK4, ITK, JAK2, JAK3, JNK3, KIT, LCK, LYN, MAPKAPK5, MET, MST4, MUSK, NEK2, NEK6, NLK, PAK1, PAK2, PAK4, PBK, PCTAIRE1, PDGFR-alpha, PDGFR-beta, PDK1, PIM1, PIM2, PKC-alpha, PKC-beta1, PKC-beta2, PKC-delta, PKC-epsilon, PKC-eta, PKC-gamma, PKC-iota, PKC-mu, PKC-theta, PKC-zeta, PLK1, PRK1, RET, ROCK2, S6K, SAK, SGK1, SGK3, SNK, SRC, SRPK2, SYK, TGFB-R1, TIE2, TSF1, TSK2, TTK, VEGF-R1, VEGF-R2, VEGF-R3, VRK1, WEE1, YES, ZAP70 especially with protein kinases like EGF-R (cell proliferation), ERBB2 (cell proliferation), PDGFR (cell proliferation), FLT3 (cell proliferation), Aurora-A (cell cycle control), Aurora-B (cell cycle control), IGF1-R (apoptosis), VEGF-R2 (angiogenesis), VEGF-R3 (angiogenesis), TIE2 (angiogenesis), EPHB4 (angiogenesis), FAK (metastasis), and SRC kinase (metastasis), there is still a great need for new therapeutic agents that inhibit these protein targets.

Thiazole derivatives described herein are a new group of protein kinase inhibitors which show differential inhibition of protein kinases, each of which can be assigned to one of the four molecular mechanisms of tumor development.

In WO 02/00649 and WO 2004/058752 quinazoline derivatives with thiazole substituents as protein kinase inhibitors are claimed.

In WO 2004/001059, WO 02/50071, WO 0062778, US 6596746, and US 6720347 azole derivatives as protein kinase inhibitors are described.

The above mentioned compounds differ from this invention in the substitution of the thiazole ring system.

The present invention relates to compounds of the general formula (I) or a salt or a physiologically functional derivative or a stereoisomer thereof, 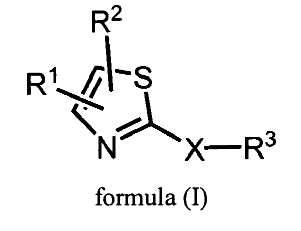 wherein
- R²: is attached at the 4- or 5-position of the thiazole ring and is hydrogen, alkyl, halogen, cyano, alkoxy, haloalkyloxy, or alkylamino;
- R¹: is attached at the 4- or 5-position of the thiazole ring and independently represents one of the following groups of the general formula (II): 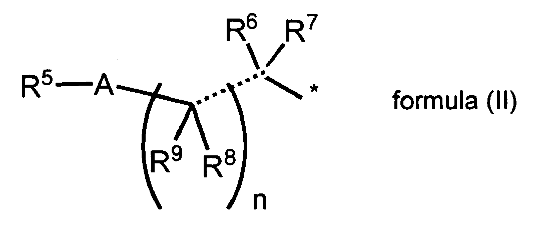
wherein
the dotted line represents a single or double bond;
- *: indicates the point of attachment to the thiazole ring;
- n: is 1, 2, or 3;
- A: independently represents a divalent linkage group selected from ←C(=O)-, ←C(=S)-, ←S(=O)-, ←S(=O)₂-, ←C(=O)O-, ←C(=O)NR¹²-, ←NR¹²C(=O)-, ←NR¹²C(=O)NR¹³-; ←NR¹²C(=O)O-, ←NR¹²NR¹³C(=O)-, ←NR¹²OC(=O)-, or ←ONR¹²C(=O)-, ←NR¹²S(=O)₂-, and where ← indicates the point of attachment to R⁵;
- R⁵: is hydrogen, alkyl, cycloalkyl, cycloalkylalkyl, aryl, arylalkyl, heteroaryl, heteroarylalkyl;
- or A and R⁵: together form an isoindol-1,3-dione-2-yl-ring which may be independently substituted by one to three substituents selected from halogen, CF₃, CHF₂, CH₂F, OCF₃, cyano, hydroxy, amino, nitro, alkoxy, alkylamino, alkyl, ethynyl, alkoxy, haloalkyloxy;
- R⁶: is hydrogen, halogen, cyano, hydroxy, amino, alkyl, alkoxy, alkylamino, cycloalkyl, haloalkyloxy, or haloalkyl;
- R⁷: is hydrogen, halogen, cyano, hydroxy, amino, alkyl, alkoxy, alkylamino, cycloalkyl, haloalkyloxy, haloalkyl, or if the dotted line represents a double bond then R⁷ is absent;
- R⁸: is hydrogen, halogen, cyano, hydroxy, amino, alkyl, alkoxy, alkylamino, cycloalkyl, haloalkyloxy, haloalkyl;
- or R⁸ and R⁶: together form a 3- to 8-membered saturated or unsaturated monocyclic ring, which may contain further heteroatoms selected from N, O or S and wherein one or more carbon atoms may be independently substituted by one to three substituents selected from halogen, CF₃, CHF₂, CH₂F, OCF₃, cyano, hydroxy, amino, nitro, alkoxy, alkylamino, alkyl, ethynyl, alkoxy, or haloalkyloxy;
- R⁹: is hydrogen, halogen, cyano, hydroxy, amino, alkyl, alkoxy, alkylamino, cycloalkyl, haloalkyloxy, haloalkyl, or if the dotted line represents a double bond then R⁹ is absent;
- R¹²: is hydrogen, alkyl, cycloalkyl, aryl, arylalkyl, or heteroaryl;
- R¹³: is hydrogen; alkyl, or cycloalkyl;

- X: independently represents a divalent linkage group selected from S, O, NR⁴, SO, or SO₂;
- R⁴: is hydrogen, alkyl, cycloalkyl, cycloalkylalkyl, or heterocyclyl;
- R³: independently represents one of the following groups, which may be independently substituted by one to three substituents R¹⁸ via the aromatic carbon atoms: 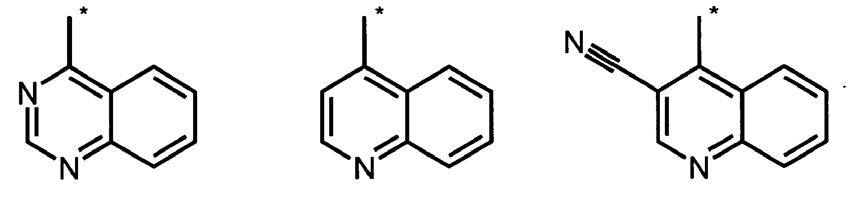 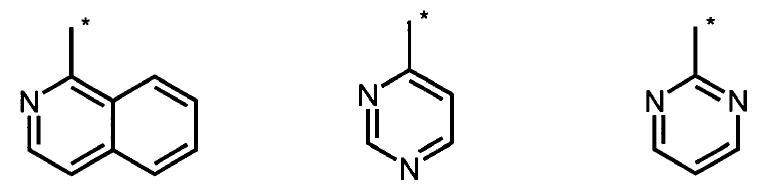 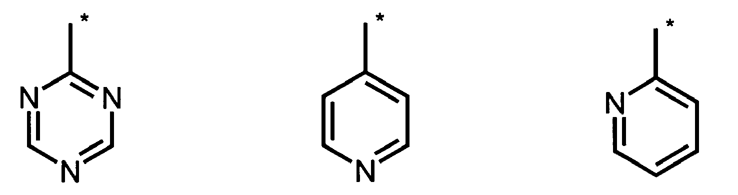 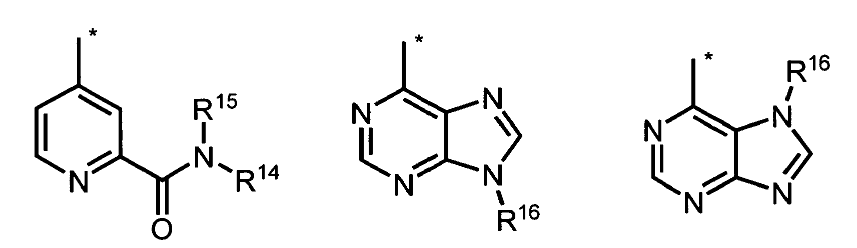 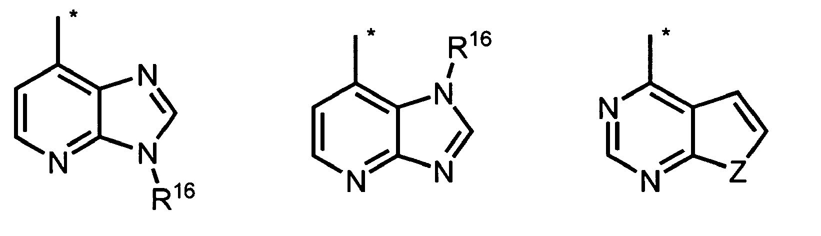 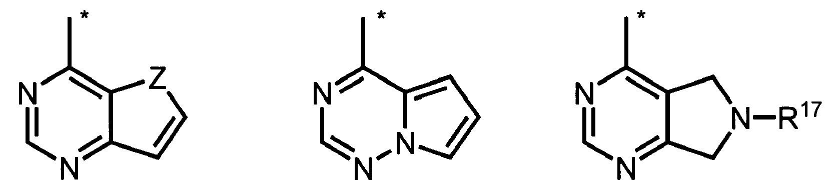
wherein
- *: indicates the point of attachment to X;
- Z: is O, NR¹⁶, or S;
- R¹⁴: is hydrogen, alkyl, cycloalkyl, heterocyclyl, or -E¹-R¹⁹;
- R¹⁵: is hydrogen or alkyl;
- R¹⁶: is hydrogen, alkyl, cycloalkyl, heterocyclyl, aryl, or heteroary;
- R¹⁷: is hydrogen or-E²-R¹⁹;
- E¹: is absent or represents a divalent linkage group selected from -O-, -N(R¹⁵)-, ←C(=O)-, ←C(=S)-, ←S(=O)-, ←S(=O)₂-, ←C(=O)O-, ←C(=O)NR¹⁶-, ←NR¹⁶C(=O)-, ←NR¹⁶C(=O)NR⁴-; ←NR¹⁶C(=O)O-, ←NR¹⁶S(=O)₂-, where ← indicates the point of attachment to the nitrogen atom in the pyridine-2-carboxylic acid amide;
- E²: is absent or represents a divalent linkage group selected from -O-, -N(R¹⁵)-, ←C(=O)-, ←C(=S)-, ←S(=O)-, ←S(=O)₂-, ←C(=O)O-, ←C(=O)NR¹⁶-, ←NR¹⁶C(=O)-, ←NR¹⁶C(=O)NR⁴-; ←NR¹⁶C(=O)O-, ←NR¹⁶S(=O)₂-, where ← indicates the point of attachment to the nitrogen atom of 6,7-dihydro-5H-pyrrolo[3,4-d]pyrimidine;
- R¹⁸: is hydrogen, halogen, hydroxy, alkoxy, haloalkyloxy, alkylamino, alkyl, cycloalkyl, haloalkyl, cyano, nitro, or -E³-R¹⁹;
- E³: is absent or represents a divalent linkage group selected from -0-, -N(R¹⁵)-, ←C(=O)-, ←C(=S)-, ←S(=O)-, ←S(=O)₂-, ←C(=O)O-, ←C(=O)NR¹⁶, ←NR¹⁶C(=O)-, ←NR¹⁶C(=O)NR⁴-; ←NR¹⁶C(=O)O-, ←NR¹⁶S(=O)₂-, where ← indicates the point of attachment to an aromatic carbon atom of the R³ residue;
- R¹⁹: is hydrogen or represents a group selected from the general formula (III) 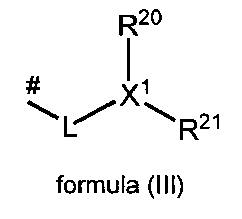 wherein
# indicates the point of attachment to E¹, or E² or E³;
L is absent or represents a divalent linkage group selected from alkylene, cycloalkylene, heterocyclylene, arylene, or heteroarylene, wherein one or more of the (-CH₂-) groups may be replaced by an oxygen or a NR¹⁵, and wherein one or more carbon atoms may be independently substituted by one or two substituents selected from halogen, hydroxy, alkoxy, haloalkyloxy, phoshonooxy, phoshonooxyalkyl;
X¹ is CH, N, or O;
R²⁰ is hydrogen, alkyl, cycloalkyl, heterocyclyl, aryl, heteroaryl, or X¹ together with R²⁰ and R²¹ form a 3- to 10-membered mono- or bicyclic, saturated, or unsaturated ring, which may contain further heteroatoms like N, O or S and wherein one or more carbon atoms may be independently substituted by R²² and each of the nitrogen atoms may be independently substituted by R²³;
R²¹ is hydrogen, alkyl, cycloalkyl, heterocyclyl, aryl, or heteroaryl;
R²² is halogen, alkoxy, alkyl, cycloalkyl, haloalkyl, haloalkyloxy, phosphonooxy, or phosphonooxyalkyl;
R²³ is hydrogen, alkyl, -CO-CH₂-OH, or -CO-CH₂-O-PO(OH)₂;
an alkyl group, if not stated otherwise, denotes a linear or branched C₁-C₆-alkyl, preferably a linear or branched chain of one to five carbon atoms, a linear or branched C₂-C₆-alkenyl or a linear or branched C₂-C₆-alkynyl group, which may be substituted by one or more substituents R';
the C₁-C₆-alkyl, C₂-C₆-alkenyl and C₂-C₆-alkynyl residue may be selected from the group comprising -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R')₃, -C₂(R')₅, -CH₂-C(R')₃, -C₃(R')₇, -C₂H₄-C(R')₃, -C₂H₄-CH=CH₂, -CH=CH-C₂H₅, -CH=C(CH₃)₂, -CH₂-CH=CH-CH₃, -CH=CH-CH=CH₂, -C₂H₄-C=CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂Hs, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH₌CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C=C-CH₃, -CH₂-C≡C-C₃H₇, -C₂H₄-C≡C-C₂H₅;

R' independently represents H, -CO₂R", -CONHR", -CR"O, -SO₂NR", -NR"-CO-haloalkyl, -NO₂, -NR"-SO₂-haloalkyl, -NR"-SO₂-alkyl, -SO₂-alkyl, -NR"-CO-alkyl, -CN, alkyl, cycloalkyl, aminoalkyl, alkylamino, alkoxy, -OH, -SH, alkylthio, hydroxyalkyl, hydroxyalkylamino, halogen, haloalkyl, haloalkyloxy, aryl, arylalkyl or heteroaryl;
R" independently represents H, haloalkyl, hydroxyalkyl, alkyl, cycloalkyl, aryl, heteroaryl or aminoalkyl; an alkylene group denotes a divalent linear or branched C₁-C₆-alkylene, preferably a linear or branched chain of one to five carbon atoms, a linear or branched C₂-C₆-alkenylene or a linear or branched C₂-C₆-alkynylene group, which may be substituted by one or more substituents R';
a cycloalkylene group denotes a divalent non-aromatic ring system containing three to eight carbon atoms, preferably four to eight carbon atoms, wherein one or more of the carbon atoms in the ring may be substituted by a group E, E being O, S, SO, SO₂, N, or NR", R" being as defined above;
a heterocyclylene group denotes a 3 to 8-membered divalent heterocyclic non-aromatic group which contains at least one heteroatom selected from O, N, and S, wherein the heterocyclylene group may be fused to another non-aromatic ring and may be substituted by one or more substituents R', wherein R' is as defined above;
an arylene group denotes an aromatic divalent group having five to fifteen carbon atoms, which may be substituted by one or more substituents R', and may be fused to another aromatic ring, where R' is as defined above;
a heteroarylene group denotes a divalent 5- or 6-membered heterocyclic group which contains at least one heteroatom selected from O, N, and S, wherein the heterocyclylene group may be fused to another aromatic ring and may be substituted by one or more substituents R', wherein R' is as defined above;
a cycloalkyl group denotes a non-aromatic ring system containing three to eight carbon atoms, preferably four to eight carbon atoms, wherein one or more of the carbon atoms in the ring may be substituted by a group E, E being O, S, SO, SO₂, N, or NR", R" being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅, morpholine-4-yl, piperazinyl, 1-alkylpiperazine-4-yl;
an alkoxy group denotes an O-alkyl group, the alkyl group being as defined above; the alkoxy group is preferably a methoxy, ethoxy, isopropoxy, t-butoxy or pentoxy group;
an alkylthio group denotes a S-alkyl group, the alkyl group being as defined above;
a haloalkyl group denotes a alkyl group which is substituted by one to five halogen atoms, the alkyl group being as defined above; the haloalkyl group is preferably a -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10''}, -C3(R¹⁰)₇, or -C₂H₄-C(R¹⁰)₃, wherein R¹⁰, R^{10'}, R^{10"} represent F, Cl, Br or I, preferably F;
a hydroxyalkyl group denotes a HO-alkyl group, the alkyl group being as defined above;
a haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes a (HO-alkyl)₂-N- group or HO-alkyl-NH- group, the alkyl group being as defined above;
an alkylamino group denotes a HN-alkyl or N-dialkyl group, the alkyl group being as defined above;
a halogen group is fluorine, chlorine, bromine, or iodine;
an aryl group denotes an aromatic group having five to fifteen carbon atoms, which may be substituted by one or more substituents R', and may be fused to another aromatic ring, where R' is as defined above; the aryl group is preferably a phenyl group, -o-C₆H₄- R', -m-C₆H₄- R', -p-C₆H₄- R', 1-naphthyl, 2-naphthyl, 1-anthracenyl or 2-anthracenyl;
a heteroaryl group denotes a 5- or 6-membered heterocyclic group which contains at least one heteroatom selected from O, N, and S, wherein the heterocyclic group may be fused to another aromatic ring; this group may be selected from a thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiadiazol-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyranyl, 3-pyranyl, 4-pyranyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, 1*H*-tetrazol-2-yl, 1*H*-tetrazol-3-yl, tetrazolyl, 2-indolyl, 3-indolyl, 2-indolinyl, 3-indolinyl, benzo[b]furanyl, benzo[b]thiophenyl, benzimidazolyl, benzothiazolyl, quinazolinyl, quinoxazolinyl, quinolinyl, tetrahydroquinolinyl, isoquinolinyl, or tetrahydroisoquinolinyl group; the heterocyclic group may be substituted by one or more substituents R', wherein R' is as defined above;
a heterocyclyl group denotes a 3 to 8-membered heterocyclic non-aromatic group which contains at least one heteroatom selected from O, N, and S, wherein the heterocyclyl group may be fused to another non-aromatic ring and may be substituted by one or more substituents R', wherein R' is as defined above;
a phosphonooxy group is -O-P(=O)(OH)₂ or a salt thereof;
a phosphonooxyalkyl group denotes an -alkyl-O-P(=O)(OH)₂ group or a salt thereof, alkyl being as defined above.

In addition, the present invention provides methods for preparing the compounds of the invention such as compounds of formula (I).

The compounds of formula (I) may be obtained *via* various methods. One possibility for the synthesis of compounds of formula (I) comprises the step of reacting a compound of formula (V), wherein R¹, R², R⁴ and X are as above defined, with a compound of formula (VI), wherein R³ is as defined above and LG comprises a leaving group e.g. Cl, Br, I, or S(=O)₂CH₃. Either nucleophilic substitution or palladium-catalyzed cross-coupling may be applied for this reaction step. If X = NR⁴, R⁴ may be added before or after addition of R³.

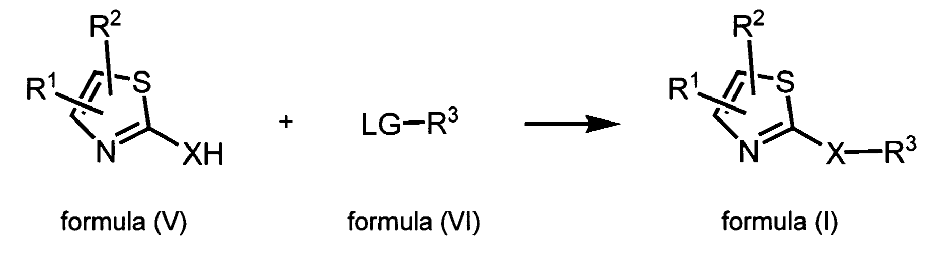

Compounds of formula (V) can be synthesized by reacting a α-bromoketone or a α-bromoaldehyde of formula (VII) with a thiourea of formula (VIII), 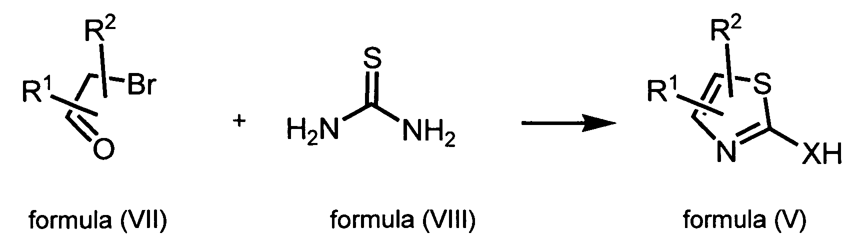 wherein R¹, R², R³ are as defined above, and X is NR⁴.

Alternatively compounds of formula (I) may be synthesized by reacting a compound of formula (VII) with a compound of formula (IX), 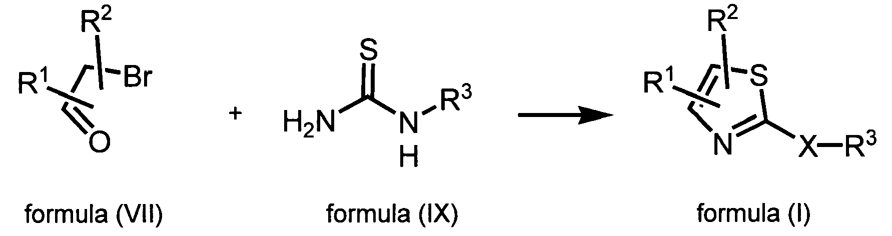 wherein R¹, R², R³ are as defined above, and X is NR⁴.

Compounds of formula (VII) may be synthesized by bromination of ketones or aldehydes described e.g. by Eriks et al., J. Med. Chem. 1992, 36, 3239-3246, or Zheng et al., J. Med. Chem. 1999, 42, 2287-2294.

An alternative way in the synthesis of a compound of formula (I), comprises the reaction of a compound of formula (X) with a nucleophile of formula (XI), 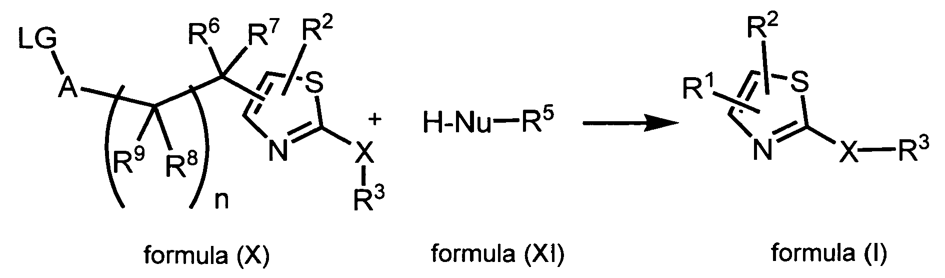 wherein R¹, R², R³, R⁵, R⁶, R⁷, R⁸, R⁹, A, and X are as defined above, LG representing a leaving group as known in the art e.g. Cl or Br, and Nu representing a nucleophilic group selected from NR¹², O, ONR¹², NR¹²O, NR¹²NR¹³.

In above formulae and in the formulae shown below, in particular in formulae (X), (XII), (XIII), (XIV), and (XX), the bond between the carbon atom having the substituents R⁸ and R⁹ and the carbon atom having the substituents R⁶ and R⁷ may be a single bond or a double bond.

An alternative way in the synthesis of a compound of formula (I), comprises the reaction of a compound of formula (XX) with a compound of formula (XXI), 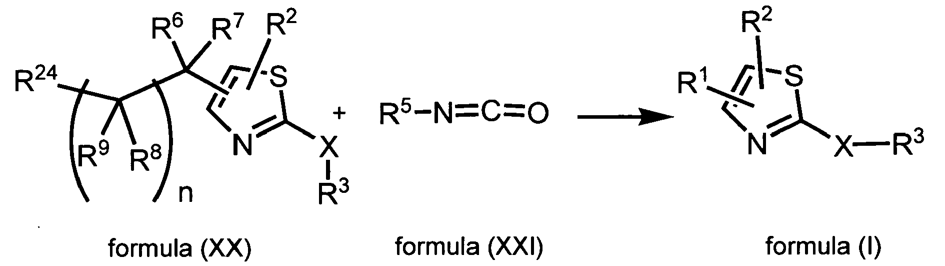 wherein R¹, R², R³, X, R⁵, R⁶, R⁷, R⁸, R⁹, being as defined above, and R²⁴ being hydroxy, amino, HNR¹²NR¹³, HONR¹³.

A compounds of the general formula (X) can be synthesized from a compound of the general formula (XII) by deprotection and activation as known in the art, 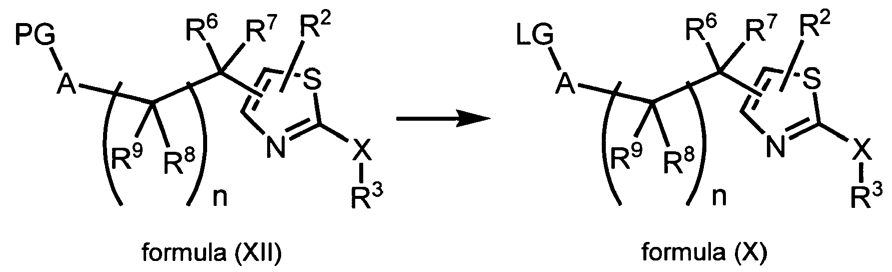 wherein R², R³, R⁵, R⁶, R⁷, R⁸, R⁹, A, and X are as defined above, PG is a protecting group for instance described in T. W. Green, P. G. M. Wuts, Protective Groups in Organic Synthesis, Wiley-Interscience, New York, 1999, and LG representing a leaving group as known in the art, e.g. Cl, Br, or I. One exemplary deprotection and activation step is the ester hydrolysis with LiOH and conversion of the resulting acid into the acid chloride by reacting it with thionyl chloride.

A compound of formula (XII) can be synthesized by reaction of a compound of formula (XIII) with a compound of formula (VI), 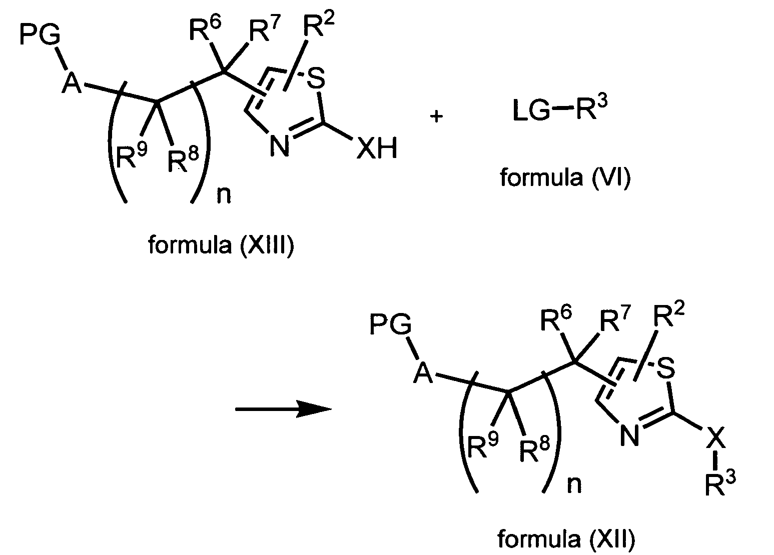 wherein PG is a protecting group and R², R³, R⁵, R⁶, R⁷, R⁸, R⁹, A, and X are as defined above, and LG representing a leaving group as known in the art.

Alternatively a compound of the formula (XII) can be synthesized by reacting a compound of the formula (XIV) with a compound of formula (IX) 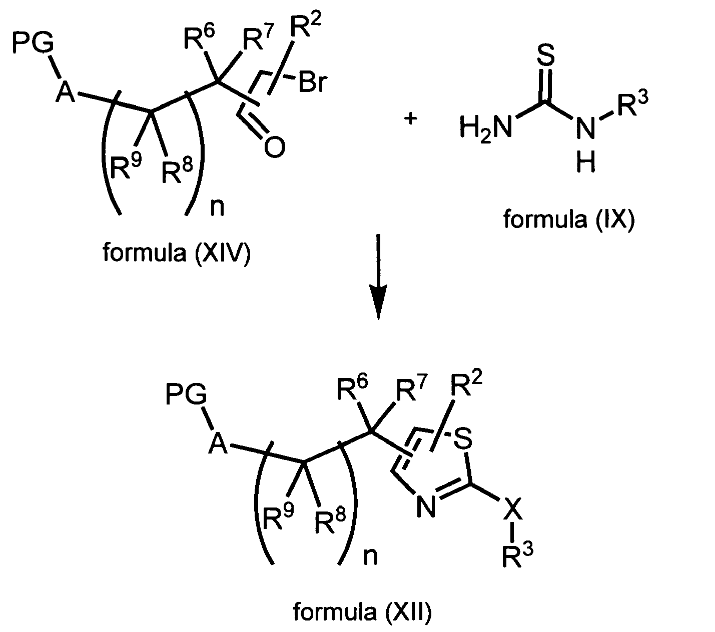 wherein PG is a protecting group and R², R³, R⁵, R⁶, R⁷, R⁸, R⁹, A are as defined above, X is NR⁴, and LG representing a leaving group.

Compounds of the general formula (I) may also be obtained by reacting a compound of the general formula (XV) with a compound of the formula (XVI). 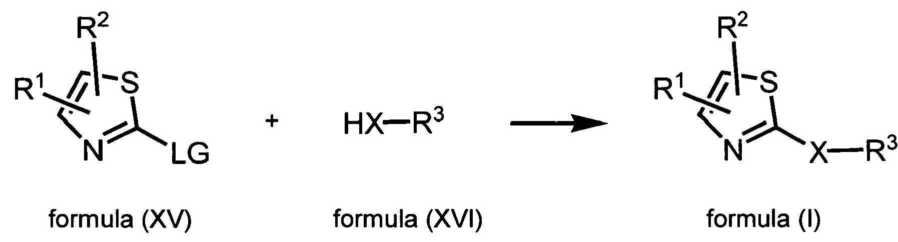 wherein R¹, R², R³, and X are as defined above, and LG representing a leaving group as known in the art. Either nucleophilic substitution or palladium-catalyzed cross-coupling may be applied for this reaction. If X = NR⁴, R⁴ may be added before or after addition of _{R}³_{.}

A preferred embodiment of the invention, are compounds of the formula (I), wherein X is NH.

A more preferred embodiment of the invention are compounds of formula (I) where R¹ is at the 4-position of the thiazole ring.

A more preferred embodiment of the invention are compounds of formula (I) where R¹ is at the 5-position of the thiazole ring.

A more preferred embodiment of the invention are compounds of formula (I) where R¹ is at the 4-position of the thiazole ring and X is NH.

A preferred embodiment of the invention, are compounds of the formula (XVII), 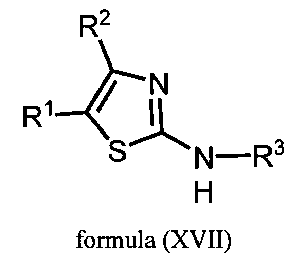 wherein R¹ is at the 5-position of the thiazole ring, X is NH and R¹, R², and R³ are as defined above.

A more preferred embodiment of the invention are compounds of formula (I) where R³ is an optionally by R¹⁸ substituted quinazoline and R¹⁸ being as defined above.

Another preferred embodiment of the invention are compounds of the formula (XVIII), 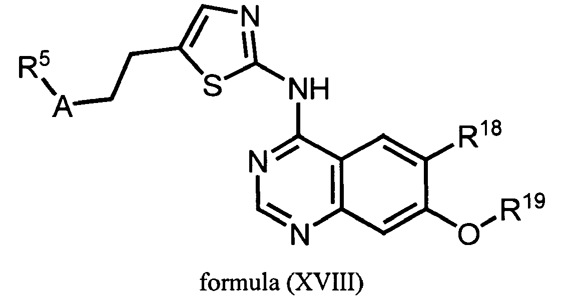 wherein A, R⁵, R¹⁸, and R¹⁹ are as defined above.

Another preferred embodiment of the invention are compounds of the formula (XIX), 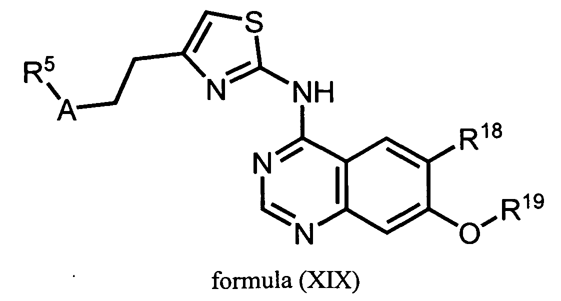 wherein A, R⁵, R¹⁸, and R¹⁹ are as defined above.

A preferred embodiment of the invention are compounds of formula (I) where A is ←C(=O)NR¹²-, and where ← indicates the point of attachment to R⁵, and R¹² is as defined above.

A preferred embodiment of the invention are compounds of formula (XVII) where A is ←C(=O)NR¹²-, and where ← indicates the point of attachment to R⁵, and R¹² is as defined above.

A preferred embodiment of the invention are compounds of formula (XVIII) where A is ←C(=O)NR¹², and where ← indicates the point of attachment to R⁵, and R¹² is as defined above.

A preferred embodiment of the invention are compounds of formula (XIX) where A is ←C(=O)NR¹²-, and where ← indicates the point of attachment to R⁵, and R¹² is as defined above.

A preferred embodiment of the invention are compounds of formula (I) where A is ←NR¹²C(=O)-, and where ← indicates the point of attachment to R⁵, and R¹² is as defined above.

A preferred embodiment of the invention are compounds of formula (XVII) where A is ←NR¹²C(=O)-, and where ← indicates the point of attachment to R⁵, and R¹² is as defined above.

A preferred embodiment of the invention are compounds of formula (XVIII) where A is ←NR¹²C(=O)-, and where ← indicates the point of attachment to R⁵, and R¹² is as defined above.

A preferred embodiment of the invention are compounds of formula (XIX) where A is ←NR¹²C(=O)-, and where ← indicates the point of attachment to R⁵, and R¹² is as defined above.

A preferred embodiment of the invention are compounds of formula (I) where A is ←NR¹²C(=O)NR¹³-, and where ← indicates the point of attachment to R⁵, and R¹² and R¹³ are as defined above.

A preferred embodiment of the invention are compounds of formula (XVII) where A is ←NR¹²C(=O)NR¹³-, and where ← indicates the point of attachment to R⁵, and R¹² and R¹³ are as defined above.

A preferred embodiment of the invention are compounds of formula (XVIII) where A is ←NR¹²C(=O)NR¹³-, and where ← indicates the point of attachment to R⁵, and R¹² and R¹³ are as defined above.

A preferred embodiment of the invention are compounds of formula (XIX) where A is ←NR¹²C(=O)NR¹³-, and where ← indicates the point of attachment to R⁵, and R¹² and R¹³ are as defined above.

In another even more preferred embodiment of the invention are compounds of formula (I) where R⁵ is an aryl or heteroaryl group which may be independently substituted by one to three halogen, CF₃, CHF₂, CH₂F, OCF₃, cyano, hydroxy, amino, nitro, alkoxy, alkylamino, alkyl, ethynyl, alkoxy, haloalkyloxy.

In another even more preferred embodiment of the invention are compounds of formula (XVII) where R⁵ is an aryl or heteroaryl group which may be independently substituted by one to three halogen, CF₃, CHF₂, CH₂F, OCF₃, cyano, hydroxy, amino, nitro, alkoxy, alkylamino, alkyl, ethynyl, alkoxy, haloalkyloxy.

In another even more preferred embodiment of the invention are compounds of formula (XVIII) where R⁵ is an aryl or heteroaryl group which may be independently substituted by one to three halogen, CF₃, CHF₂, CH₂F, OCF₃, cyano, hydroxy, amino, nitro, alkoxy, alkylamino, alkyl, ethynyl, alkoxy, haloalkyloxy.

In another even more preferred embodiment of the invention are compounds of formula (XIX) where R⁵ is an aryl or heteroaryl group which may be independently substituted by one to three halogen, CF₃, CHF₂, CH₂F, OCF₃, cyano, hydroxy, amino, nitro, alkoxy, alkylamino, alkyl, ethynyl, haloalkyloxy.

In another even more preferred embodiment of the invention are compounds of formula (XVIII) where R¹⁸ is methoxy.

In another even more preferred embodiment of the invention are compounds of formula (XIX) where R¹⁸ is methoxy.

In another even more preferred embodiment of the invention are compounds of formula (XVIII) where R¹⁸, is methoxy and R¹⁹ is selected from 2-morpholin-4-yl-ethyl, 3-morpholin-4-yl-propyl, 2-(4-methyl-piperazin-1-yl)-ethyl, 3-(4-methyl-piperazin-1-yl)-propyl, 2-pyrrolidin-1-yl-ethyl, 3-pyrrolidin-1-yl-propyl, 2-(1-methyl-piperidin-4-yl)-ethyl, 3-(1-methyl-piperidin-4-yl)-propyl, 2-dimethylamino-ethyl, 3-dimethylamino-propyl, (3-morpholin-4-yl)-2-hydroxy-propyl, [3-(4-methyl-piperazin-1-yl)]-2-hydroxy-propyl, (3-pyrrolidin-1-yl)-2-hydroxy-propyl, [3-(1-methyl-piperidin-4-yl)]-2-hydroxy-propyl, 2-(3-hydroxy-pyrrolidin)-1-yl-ethyl, 3-(3-hydroxy-pyrrolidin)-1-yl-propyl, 2-(2-hydroxymethyl-pyrrolidin)-1-yl-ethyl, 3-(2-hydroxymethyl-pyrrolidin)-1-yl-propyl, and 2-methoxy-ethyl.

In another even more preferred embodiment of the invention are compounds of formula (XIX) where R¹⁸ is methoxy and R¹⁹ is selected from 2-morpholin-4-yl-ethyl, 3-morpholin-4-yl-propyl, 2-(4-methyl-piperazin-1-yl)-ethyl, 3-(4-methyl-piperazin-1-yl)-propyl, 2-pyrrolidin-1-yl-ethyl, 3-pyrrolidin-1-yl-propyl, 2-(1-methyl-piperidin-4-yl)-ethyl, 3-(1-methyl-piperidin-4-yl)-propyl, 2-dimethylamino-ethyl, 3-dimethylamino-propyl, (3-morpholin-4-yl)-2-hydroxy-propyl, [3-(4-methyl-piperazin-1-yl)]-2-hydroxy-propyl, (3-pyrrolidin-1-yl)-2-hydroxy-propyl, [3-(1-methyl-piperidin-4-yl)]-2-hydroxy-propyl, 2-(3-hydroxy-pyrrolidin)-1-yl-ethyl, 3-(3-hydroxy-pyrrolidin)-1-yl-propyl, 2-(2-hydroxymethyl-pyrrolidin)-1-yl-ethyl, 3-(2-hydroxymethyl-pyrrolidin)-1-yl-propyl, and 2-methoxy-ethyl.

In another even more preferred embodiment of the invention are compounds of formula (XVIII) where A is C(=O)NR¹² or NR¹²C(=O), and R¹⁸ is methoxy, and R¹⁹ is selected from 2-morpholin-4-yl-ethyl, 3-morpholin-4-yl-propyl, 2-(4-methyl-piperazin-1-yl)-ethyl, 3-(4-methyl-piperazin-1-yl)-propyl, 2-pyrrolidin-1-yl-ethyl, 3-pyrrolidin-1-yl-propyl, 2-(1-methyl-piperidin-4-yl)-ethyl, 3-(1-methyl-piperidin-4-yl)-propyl, 2-dimethylamino-ethyl, 3-dimethylamino-propyl, (3-morpholin-4-yl)-2-hydroxy-propyl, [3-(4-methyl-piperazin-1-yl)]-2-hydroxy-propyl, (3-pyrrolidin-1-yl)-2-hydroxy-propyl, [3-(1-methyl-piperidin-4-yl)]-2-hydroxy-propyl, 2-(3-hydroxy-pyrrolidin)-1-yl-ethyl, 3-(3-hydroxy-pyrrolidin)-1-yl-propyl, 2-(2-hydroxymethyl-pyrrolidin)-1-yl-ethyl, 3-(2-hydroxymethyl-pyrrolidin)-1-yl-propyl, and 2-methoxy-ethyl.

In another even more preferred embodiment of the invention are compounds of formula (XIX) where A is C(=O)NR¹² or NR¹²C(=O), and R¹⁸ is methoxy, and R¹⁹ is selected from 2-morpholin-4-yl-ethyl, 3-morpholin-4-yl-propyl, 2-(4-methyl-piperazin-1-yl)-ethyl, 3-(4-methyl-piperazin-1-yl)-propyl, 2-pyrrolidin-1-yl-ethyl, 3-pyrrolidin-1-yl-propyl, 2-(1-methyl-piperidin-4-yl)-ethyl, 3-(1-methyl-piperidin-4-yl)-propyl, 2-dimethylamino-ethyl, 3-dimethylamino-propyl, (3-morpholin-4-yl)-2-hydroxy-propyl, [3-(4-methyl-piperazin-1-yl)]-2-hydroxy-propyl, (3-pyrrolidin-1-yl)-2-hydroxy-propyl, [3-(1-methyl-piperidin-4-yl)]-2-hydroxy-propyl, 2-(3-hydroxy-pyrrolidin)-1-yl-ethyl, 3-(3-hydroxy-pyrrolidin)-1-yl-propyl, 2-(2-hydroxymethyl-pyrrolidin)-1-yl-ethyl, 3-(2-hydroxymethyl-pyrrolidin)-1-yl-propyl, and 2-methoxy-ethyl.

Exemplary compounds of formula (I) of the present invention include, but are not limited to, the following:
3-Fluoro-N-(2-{2-[6-methoxy-7-(3-pyrrolidin-1-yl-propoxy)-quinazolin-4-ylamino]-thiazol-5-yl}-ethyl)-benzamide (1),
N-(2-{2-[6-Methoxy-7-(3-pyrrolidin-1-yl-propoxy)-quinazolin-4-ylamino]-thiazol-5-yl}-ethyl)-benzamide (2),
3-[2-(6,7-Dimethoxy-quinazolin-4-ylamino)-thiazol-5-yl]-propionic acid methyl ester (3),
3-[2-(6,7-Dimethoxy-quinazolin-4-ylamino)-thiazol-5-yl]-propionic acid (4),
N-(3-Fluoro-phenyl)-3-{2-[6-methoxy-7-(3-pyrrolidin-1-yl-propoxy)-quinazolin-4-ylamino]-thiazol-5-yl}-propionamide (5),
2-(2-{2-[6-Methoxy-7-(3-pyrrolidin-1-yl-propoxy)-quinazolin-4-ylamino]-thiazol-4-yl}-ethyl)-isoindole-1,3-dione (6).

The compounds of the formula (I) to be used according to the invention can form salts with inorganic or organic acids or bases. Examples of pharmaceutically acceptable salts comprise without limitation non-toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-*p*-sulfate derived from p-toluenesulfonic acid and others.

Salts of phosphonoxy- and phosphonoxyalkyl groups may be those formed with alkali metal ions e.g. sodium or potassium, or those formed with alkaline earth metal ions e. g. calcium or magnesium, or those formed with zinc ions.

Such salts of the compounds of the present invention may be anhydrous or solvated. Such salts can be produced by methods known to someone skilled in the art and described in the prior art.

The compounds according to the invention and medicaments prepared therewith are generally useful for treating, relieving, and/or preventing cell proliferation disorders, for the treatment or prophylaxis of immunological diseases and conditions (as for instance inflammatory diseases, neuroimmunological diseases, autoimmune diseases or other).

The compounds of the present invention are useful for treating, relieving, and/or preventing diseases which are caused by malignant cell proliferation. Therefore the compounds according to the invention and medicaments prepared therewith are generally useful for regulating cell activation, cell proliferation, cell survival, cell differentiation, cell cycle, cell maturation and cell death or to induce systemic changes in metabolism such as changes in sugar, lipid or protein metabolism. They can also be used to support cell generation poiesis, including blood cell growth and generation (prohematopoietic effect) after depletion or destruction of cells, as caused by, for example, toxic agents, radiation, immunotherapy, growth defects, malnutrition, malabsorption, immune dysregulation, anemia and the like or to provide a therapeutic control of tissue generation and degradation, and therapeutic modification of cell and tissue maintenance and blood cell homeostasis.

These diseases and conditions include but are not limited to cancer as hematological tumors (e.g. leukemia, myeloma), or lymphomas (e.g. Hodgkin's and non-Hodgkin's lymphomas), or solid tumors (for example breast, prostate, liver, bladder, lung, esophageal, stomach, colorectal, genitourinary, gastrointestinal, skin, pancreatic, brain, uterine, colon, head and neck, cervical, and ovarian, melanoma, astrocytoma, small cell lung cancer, glioma, basal and squameous cell carcinoma, sarcomas as Kaposi's sarcoma and osteosarcoma).

Other aspects of the present invention relate to thiazole derivatives as new pharmaceutically active agents, especially for the preparation of a pharmaceutical composition for treating, relieving, and/or preventing diseases which are cured, relieved, or prevented by the inhibition of one or several kinases and/or phosphatases.

In another more preferred embodiment of the invention the compounds of formula (I), formula (XVII), formula (XVIII), and formula (XIX) may be used for treating and/or preventing diseases by inhibition of one ore more kinases like: Aurora-A, Aurora-B, EGF-R, ERBB2, PDGFR, FLT3, IGF1-R, VEGF-R2, VEGF-R3, EPHB4, TIE2, FAK, and SRC.

The compounds according to the invention or a pharmaceutically acceptable salt or physiologically functional derivative or a stereoisomer thereof if desired with appropriate adjuvants and additives for the production of a medicament for the treatment or prevention of a disease characterized by hyperproliferation of keratinocytes and/or T cells, especially inflammatory disorders and immune disorders, preferably selected from the group consisting of Addison's disease, alopecia areata, Ankylosing spondylitis, haemolytic anemia (anemia haemolytica), pernicious anemia (anemia perniciosa), aphthae, aphthous stomatitis, arthritis, arteriosclerotic disorders, osteoarthritis, rheumatoid arthritis, aspermiogenese, asthma bronchiale, auto-immune asthma, auto-immune hemolysis, Bechet's disease, Boeck's disease, inflammatory bowel disease, Burkitt's lymphoma, Crohn's disease, chorioiditis, colitis ulcerosa, Coeliac disease, cryoglobulinemia, dermatitis herpetiformis, dermatomyositis, insulin-dependent type I diabetes, juvenile diabetes, idiopathic diabetes insipidus, insulin-dependent diabetes mellisis, autoimmune demyelinating diseases, Dupuytren's contracture, encephalomyelitis, encephalomyelitis allergica, endophthalmia phacoanaphylactica, enteritis allergica, autoimmune enteropathy syndrome, erythema nodosum leprosum, idiopathic facial paralysis, chronic fatigue syndrome, febris rheumatica, glomerulo nephritis, Goodpasture's syndrome, Graves' disease, Harnman-Rich's disease, Hashimoto's disease, Hashimoto's thyroiditis, sudden hearing loss, sensoneural hearing loss, hepatitis chronica, Hodgkin's disease, haemoglobinuria paroxysmatica, hypogonadism, ileitis regionalis, iritis, leucopenia, leucemia, lupus erythematosus disseminatus, systemic lupus erythematosus, cutaneous lupus erythematosus, lymphogranuloma malignum, mononucleosis infectiosa, myasthenia gravis, traverse myelitis, primary idiopathic myxedema, nephrosis, ophthalmia symphatica, orchitis granulomatosa, pancreatitis, pemphigus, pemphigus vulgaris, polyarteritis nodosa, polyarthritis chronica primaria, polymyositis, polyradiculitis acuta, psoriasis, purpura, pyoderma gangrenosum, Quervain's thyreoiditis, Reiter's syndrome, sarcoidosis, ataxic sclerosis, progressive systemic sclerosis, scleritis, sclerodermia, multiple sclerosis, sclerosis disseminata, acquired spenic atrophy, infertility due to antispermatozoan antibodies, thrombocytopenia, idiopathic thrombocytopenia purpura, thymoma, acute anterior uveitis, vitiligo, AIDS, HIV, SCID and Epstein Barr virus associated diseases such as Sjorgren's syndrome, virus (AIDS or EBV) associated B cell lymphoma, parasitic diseases such as Leishmania, and immunesuppressed disease states such as viral infections following allograft transplantations, AIDS, cancer, chronic active hepatitis diabetes, toxic chock syndrome and food poisoning.

"Treatment" according to the present invention is intended to mean complete or partial healing of a disease, prevention of a disease, or alleviation of a disease or stop of progression of a given disease.

The compounds of the present invention can further be used for diseases that are caused by protozoal infestations in humans and animals.

The compounds of the present invention can further be used for viral infections.

Furthermore, the invention relates to a method of treatment or prevention of diseases which comprises the administration of an effective amount of compounds of the formula (I), or a pharmaceutically acceptable salt or physiologically functional derivative or a stereoisomer thereof.

The compounds of the according invention and their pharmacologically acceptable salts can be administered to animals, preferably to mammals, and in particular to humans as therapeutics per se, as mixtures with one another or in the form of pharmaceutical preparations which allow enteral (e.g. oral) or parenteral use and which as active constituent contain an effective dose of at least one compound of the formula (I), or a salt thereof, in addition to customary pharmaceutically innocuous excipients and additives.

The production of medicaments containing the compounds of formula (I), according to the invention and their application can be performed according to well-known pharmaceutical methods.

The invention also provides a pharmaceutical composition comprising a compound of formula (I), in free form or in the form of pharmaceutically acceptable salts and physiologically functional derivatives, together with a pharmaceutically acceptable diluent or carrier therefore.

The term "physiologically functional derivative" as used herein refers to compounds which are not pharmaceutically active themselves but which are transformed into their pharmaceutical active form in vivo, i.e. in the subject to which the compound is administered. Examples of physiologically functional derivatives are prodrugs.

Prodrugs of the compounds of the present invention include but are not limited to: esters, which are transformed in vivo into the corresponding active alcohol, esters, which are transformed in vivo into the corresponding active acid, imines, which are transformed in vivo into the corresponding amines, imines which are metabolized in vivo into the corresponding active carbonyl derivative (e.g. aldehyde or ketone), 1-carboxy-amines, which are decarboxylated in vivo into the active amine, phosphoryloxy-compounds, which are dephosporylated in vivo by phosphateases into the active alcohols, and amides which are metabolized into the corresponding active amine or acid respectively.

The term "stereoisomere" as used herein refers to compound with at least one stereogenic center, which may be R- or S-configurated. It has to be understood, that in compounds with more than one stereogenic center each of which independently from each other can be R- or S-configurated. Examples of such compound are the compounds of formula (I), where R⁶ is not R⁷ or where R⁸ is not R⁹ and the dotted line in formula (II) is a single bond. The term "stereoisomere" as used herein also refers to salts of the compounds herein described with optically active acids or bases.

While the compounds according to the invention for use in therapy may be administered in the form of the raw chemical compound, it is preferred to introduce the active ingredient, optionally in the form of a physiologically acceptable salt in a pharmaceutical composition together with one or more adjuvants, excipients, carriers, buffers, diluents, and/or other customary pharmaceutical auxiliaries.

In a preferred embodiment, the invention provides medicaments comprising compounds according to the invention, or a pharmaceutically acceptable salt or physiologically functional derivative or a stereoisomer thereof, together with one or more pharmaceutically acceptable carriers thereof, and, optionally, other therapeutic and/or prophylactic ingredients. The carrier(s) must be "acceptable" in the sense of being compatible with the other ingredients of the formulation and not harmful to the recipient thereof.

A medicament of the invention may be those suitable for oral, rectal, bronchial, nasal, topical, buccal, sub-lingual, transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

For preparing a medicament from a compounds of formula (I), pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or an encapsulating material.

In powders, the carrier is a finely divided solid which is in a mixture with the finely divided active component. In tablets, the active component is mixed with the carrier having the necessary binding capacity in suitable proportions and compacted in the shape and size desired. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify. Compositions suitable for vaginal administration may be presented as pessaries, tampons, creams, gels, pastes, foams or sprays containing in addition to the active ingredient such carriers as are known in the art to be appropriate. Liquid preparations include solutions, suspensions, and emulsions, for example, water or water-propylene glycol solutions. For example, parenteral injection liquid preparations can be formulated as solutions in aqueous polyethylene glycol solution.

The compounds according to the present invention may be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavors, stabilizing and thickening agents, as desired. Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

In an especially preferred embodiment of the present invention the medicament is applied topically. This reduces possible side effects and limits the necessary treatment to those areas affected.

Preferably the medicament is prepared in form of an ointment, a gel, a plaster, an emulsion, a lotion, a foam, a cream of a mixed phase or amphiphilic emulsion system (oil/water-water/oil mixed phase), a liposome, a transfersome, a paste or a powder.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Compositions suitable for topical administration in the mouth include lozenges comprising the active agent in a flavored base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form. Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co. Easton, Pa.).

Pharmaceutical compositions can also contain two or more compounds of the formula (I), or their pharmacologically acceptable salts and also other therapeutically active substances.

Thus, the compounds of the present invention can be used in the form of one compound alone or in combination with other active compounds - for example with medicaments already known for the treatment of the aforementioned diseases, whereby in the latter case a favorable additive, amplifying effect is noticed.

Thus, the compounds of the present invention can be used in combination with radiation therapy, or in combination with radiation therapy and other active compounds, already known for the treatment of the aforementioned diseases, whereby a favorable additive or amplifying effect is noticed.

To prepare the pharmaceutical preparations, pharmaceutically inert inorganic or organic excipients can be used. To prepare pills, tablets, coated tablets and hard gelatin capsules, for example, lactose, corn starch or derivatives thereof, talc, stearic acid or its salts, etc. can be used. Excipients for soft gelatin capsules and suppositories are, for example, fats, waxes, semi-solid and liquid polyols, natural or hardened oils etc. Suitable excipients for the production of solutions and syrups are, for example, water, sucrose, invert sugar, glucose, polyols etc. Suitable excipients for the production of injection solutions are, for example, water, alcohols, glycerol, polyols or vegetable oils.

The dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 1 to 100 mg/kg animal body weight preferably 1 to 50 mg/kg. Suitable dosage rates for larger mammals, for example humans, are of the order of from about 10 mg to 3 g/day, conveniently administered once, in divided doses 2 to 4 times a day, or in sustained release form.

The following examples are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims.

### Examples

Abbreviations: min, minute(s); h, hour(s); r.t., room temperature; TLC, thin layer chromatography; XANTPHOS, 9,9-dimethyl-4,5-bis(diphenylphosphino)xanthene; dba, dibenzylideneacetone; DME, 1,2-dimethoxyethane; DIEA, *N,N* diisopropylethylamine; EDC, 1-(3-dimethylaminopropyl)-3-ethylcarbodiimide; No, number.

Preparative HPLC-MS: Waters 600 Multisolvent Delivery System with peparative pump heads. 2000 µl or 5000 µl Sample loop. Column, Waters X-Terra RP18, 7 µm, 19 x 150 mm with X-Terra RP 18 guard cartridge 7 µm, 19 x 10 mm; used at flow rate 20 ml/min or YMC ODS-A, 120 A, 40 x 150 mm with X-Terra RP18 guard cartridge 7 µm, 19 x 10 mm; used at flow rate 50 ml/min. Make-up solvent: MeCN - H₂O - HCO₂H 80 : 20 : 0.05 (v:v:v). Eluent A, H₂O + 0.1% HCO₂H; eluent B, MeCN. Different linear gradients from 5 - 100% eluent B, adapted to sample. Injection volume: 500 µl - 2000 µl depending on sample.

### Syntheses of 4-chloroquinazolines with an alkylamino sidechain:

Step 1. To a solution of methyl vanillate or methyl isovanillate (7.29 g, 40 mmol) in dimethylformamide (25 mL), potassium carbonate (8.29 g, 60 mmol) and benzyl bromide (5.26 mL, 44 mmol) were added. The mixture was heated to 100°C for 3 h. After cooling to r.t., water was added and the product was extracted several times with ethyl acetate. The combined organic phases were washed with water and brine. After drying over Na₂SO₄, the solvent was removed to yield methyl 4-benzyloxy-3-methoxybenzoate or methyl 3-benzyloxy-4-methoxybenzoate, respectively, quantitatively, which was used without further purification.

Step 2. Crude material of step 1 (40.0 mmol) was converted into methyl 4-benzyloxy-5-methoxy-2-nitrobenzoate or methyl 5-benzyloxy-4-methoxy-2-nitrobenzoate, respectively, in 91-94% yield as described in US 02/0026052 A1, page 51, reference example 15.

Step 3. In a 1 L Schlenk flask filled with argon, product of step 2 (36.6 mmol) and palladium on charcoal (1.17 g, 10% Pd, 1.1 mmol Pd) were combined and tetrahydrofuran (250 mL) was added. The argon was replaced with hydrogen (1 bar), and the mixture was vigorously stirred at r.t. until completion of the reaction. The palladium was separated by filtration through a pad of celite and the solvent was removed to obtain methyl 2-amino-4-hydroxy-5-methoxybenzoate or methyl 2-amino-5-hydroxy-4-methoxybenzoate, respectively, quantitatively, which, again, was used without further purification.

Step 4. A mixture of formamide (29 mL), ammonium formate (3.41 g, 54 mmol) and crude material of step 3 (36.0 mmol) was heated to 140°C for 4 h. After cooling to r.t., water (75 mL) was added. After stirring for 1 h, the precipitated 7-hydroxy-6-methoxy-3,4-dihydroquinazolin-4-one or 6-hydroxy-7-methoxy-3,4-dihydroquinazolin-4-one, respectively, was filtered off, washed with water and dried (76-85%).

Step 5. A mixture of product step 4 (30.5 mmol), acetic anhydride (21.5 mL, 229 mmol) and pyridine (4.9 mL, 61 mmol) was heated to 100°C for 4 h. After cooling to r.t., ice water (200 mL) was added and the mixture was vigorously stirred for 1 h. The precipitated 7-acetoxy-6-methoxy-3,4-dihydroquinazolin-4-one or 6-acetoxy-7-methoxy-3,4-dihydroquinazolin-4-one, respectively, was filtered off, washed with water and dried (93-96%).

Step 6. Product step 5 (8.54 mmol) was converted into 4-chloro-7-hydroxy-6-methoxyquinazoline or 4-chloro-6-hydroxy-7-methoxyquinazoline, respectively, (58-95%) by reacting them with thionyl chloride (12 mL) and DMF (0.3 mL) at 85 °C for 1.5 h. Excess thionyl chloride was removed by distillation. Traces of thionyl chloride were removed by aceotropic distillation wit toluene (two times). Alternatively the products step 5 can be converted into the chlorides by reacting them with a mixture of POCl₃ and PCl₅. The acetyl groups were removed by hydrolysis with ammonium hydroxide (5 mL, 28-30 wt%) in dioxane / water (100 mL / 20 mL) at 0 °C to r.t.

Step 7. General procedure 1: Di-*tert*-butyl azodicarboxylate (0.478 g, 2.08 mmol) was added portionwise to a mixture of product step 6 (1.66 mmol), 3-(4-methylpiperazin-1-yl)-propan-1-ol (synthesis described below, 0.276 g, 1.74 mmol), and triphenylphosphine (0.544 g, 2.08 mmol) in dichloromethane (20 mL) at r.t.. If necessary, further alcohol was added. After stirring for 2 h, the solution was concentrated to 10 mL, mounted on silica and chromatographed (gradient, dichloromethane to dichloromethane : methanol = 3:2) to obtain the desired ethers (~73%).

### Synthesis of 4-chloro-6-methoxy-7-[3-(4-methylpiperazin-1-yl)propoxy]quinazoline:

The compound was synthesized according to general procedure 1 from 4-chloro-7-hydroxy-6-methoxyquinazoline. LC/ESI-MS: m/*z* =351 [M+H].

### Synthesis of 4-chloro-7-methoxy-6-[3-(4-methylpiperazin-1-yl)propoxy]quinazoline:

The compound was synthesized according to general procedure 1 from 4-chloro-6-hydroxy-7-methoxyquinazoline. LC/ESI-MS: *m*/*z* = 351 [M+H].

### Synthesis of 4-chloro-6-methoxy-7-(3-pyrrolidin-1-yl-propoxy)-quinazoline:

The compound was synthesized according to general procedure 1 from 4-chloro-7-hydroxy-6-methoxyquinazoline. LC/ESI-MS: *m*/*z* = 322 [M+H].

### General Procedure 2 (Swern Oxidation) GP2:

Oxalyl chloride (4.80g, 37.8 mmol, 1.5 eq.)was dissolved in an inert atmosphere in dichloromethane (60 mL). DMSO (5.91 g, 75.6 mmol, 3 eq.) in dichloromethane (20 mL) was added drop wise at -78 °C. The temperature was allowed to reach -50 °C within 15 min. The alcohol (25.2 mmol, 1.0 eq.) was added as a solution in dichloromethane (60 mL) at -78 °C. The temperature was allowed to reach -50 °C within 15 to 45 min. After cooling to -78 °C triethylamine (17.9 g, 177 mmol, 7.0 eq.) was added. The dry ice acetone bath was replaced by an ice bath. The reaction mixture was kept for about 1 h at 0 °C. The reaction was quenched through addition of water (80 mL) and dichloromethane (300 mL). The phases were separated, the organic phase was washed with water (2 x 80 mL) and dried with Na₂SO₄. After filtration the solvent was removed in vacuo. The aldehyde was subsequently used without further purification.

### General Procedure 3 (Rosenmund reduction) GP3:

The acid chloride (1.0 eq.) and 2,6-lutidine (1.1 eq.) were dissolved in dry THF in an inert atmosphere. Pd on charcoal (10% Pd, 0.2 eq.) was added portion wise. The inert atmosphere was replaced by an H₂-atmosphere. After 15 h the reaction mixture was filtered through a pad of celite (1.5 cm). THF was reduced in vacuo. The residue was separated between dichloromethane (400 mL) and HC1 (1 M, 150 mL). The phases were separated, the organic layer was washed with 1 M HC1 (150 ml) and water (100 mL) and dried with Na₂SO₄. After filtration the solvent was removed in vacuo. The aldehyde was subsequently used without further purification.

### General Procedure 4 (bromination) GP4:

The crude aldehyde or the ketone (60.7 mmol) was dissolved in chloroform (100 mL).

Bromine (60.7 mmol, 1.0 eq.) in chloroform (50 mL) was added in an inert atmosphere. After 4 to 15 h the reaction was quenched by addition of water (80 mL) and DCM (300 mL) and neutralized by addition of NaHCO₃ aqueous saturated solution. The layers were separated. The organic layer was washed with 1 M HC1 (100 mL) and water (150 mL) and dried with Na₂SO₄. After filtration the solvent was removed in vacuo. The bromide was subsequently used without further purification.

### General Procedure 5 (thiazol synthesis) GP5:

The crude bromide (44 mmol, 1.0 eq.) was dissolved in methanol, ethanol or isopropanol (80 ml) . Thiourea (44 mmol, 1.0 eq.) was added in an inert atmosphere. The reaction was stirred at 60 to 80 °C for 10 to 15 h after which it was quenched by addition of water (200 mL) and saturated aqueous NaHCO₃-solution (150 mL). The product was extracted with dichloromethane (4 x 180 mL). The combined organic phases were extracted with 1 M HCl (4 x 125 mL). The water layers were combined and neutralized with solid NaOH and NaHCO₃. The product was extracted with dichloromethane (4 x 200 mL). The combined organic phases were dried with Na₂SO₄. The solvent was removed in vacuo and the product dried at an oil pump.

### Synthesis of N-[2-(2-Amino-thiazol-5-yl)-ethyl]-3-fluoro-benzamide:

N-[2-(2-Amino-thiazol-5-yl)-ethyl]-3-fluoro-benzamide was synthesized from 3-Fluoro-N-(4-hydroxy-butyl)-benzamide according to GP2, GP4 and GP5. LC/ESI-MS: m/z = 266 [M+H].

### Synthesis of N-[2-(2-Amino-thiazol-5-yl)-ethyl]-benzamide:

N-[2-(2-Amino-thiazol-5-yl)-ethyl]-benzamide was synthesized from N-(4-Hydroxybutyl)-benzamide according to GP2, GP4, and GP5. LC/ESI-MS: *m*/*z* = 248 [M+H].

### Synthesis of 3-(2-Amino-thiazol-5-yl)-propionic acid methyl ester:

3-(2-Amino-thiazol-5-yl)-propionic acid methyl ester was synthesized from 4-Chlorocarbonyl-butyric acid methyl ester according to GP3, GP4, and GP5. LC/ESI-MS: m/z = 187 [M+H].

### Synthesis of 3-(2-Amino-thiazol-5-yl)-N-(3-fluoro-phenyl)-propionamide:

3-(2-Amino-thiazol-5-yl)-N-(3-fluoro-phenyl)-propionamide was synthesized from 3-(2-Amino-thiazol-5-yl)-propionic acid methyl ester by Boc-protection of the 2-amino function followed by ester hydrolysis with LiOH, peptide coupling with 3-fluoro aniline and Boc-deprotection. LC/ESI-MS: m/z = 266[M+H].

### Synthesis of 2-[2-(2-Amino-thiazol-4-yl)-ethyl]-isoindole-1,3-dione

2-[2-(2-Amino-thiazol-4-yl)-ethyl]-isoindole-1,3-dione was synthesized from 2-(3-Oxobutyl)-isoindole-1,3-dione according to GP4 and GP5. LC/ESI-MS: *m*/*z* = 220 [M+H].

### Syntheses of screening compounds:.

### General Procedure 6 (Pd-catalyzed amination):

In a Schlenk flask under an argon atmosphere, finely ground water-free potassium phosphate (1.1 equiv) is added to a mixture of the appropriate 2-aminothiazole (0.2 mmol) and the appropriate 4-chloroquinazoline (0.2 mmol) followed by dry dioxane (1 mL). After addition of 4,5-bis(diphenylphosphino)-9,9-dimethylxanthene (xantphos, 12 µmol) and tris(dibenzylideneacetone)dipalladium (4 µmol, 4 mol-% of Pd), the flask is sealed and heated to 100°C overnight with stirring. The mixture is allowed to cool to r.t. and the product is obtained by filtration and by chromatography respectively.

### General Procedure 7 (peptide coupling HBTU):

The acid (0.55 mmol) was dissolved in dry DMF (5 mL) under an inert atmosphere. HBTU (0.55 mmol), the amine (0.55 mmol), and the base triethylamine (1.5 eq., 0.83 mmol) were added. The reaction was stirred at r.t. or at 80 °C until completion.

By following the methods described above, the compounds set out in the following table were prepared.

| Compound No. | Compound Structure | LC/ESI-MS: [M+H] *m*/*z* = | General Procedure |
|---|---|---|---|
| 1 | 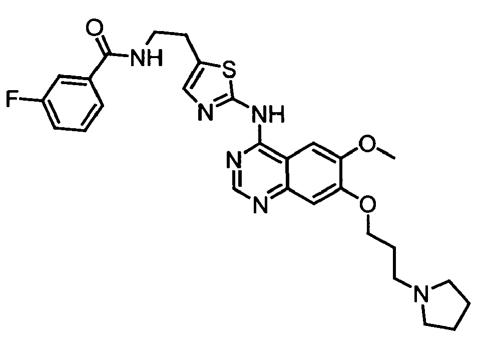 | 551 | 6 |
| 2 | 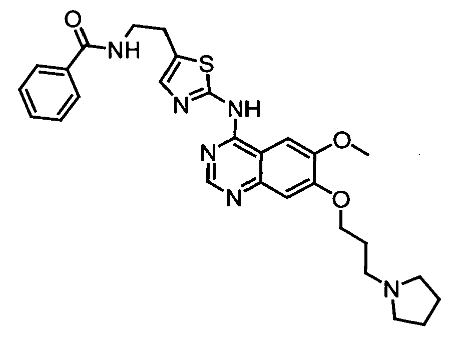 | 533 | 6 |
| 3 | 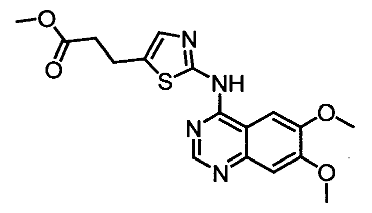 | 375 | 6 |
| 4 | 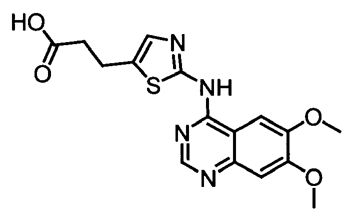 | 361 | from 3 |
| 5 | 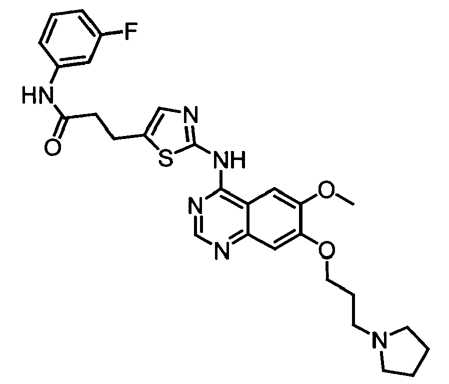 | 551 | 6 |
| 6 | 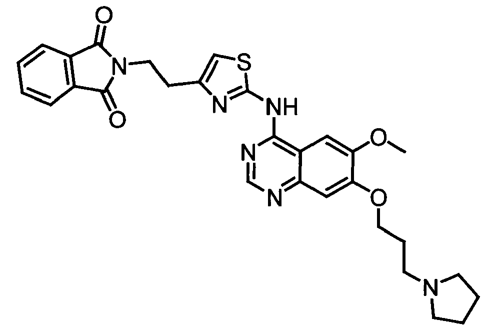 | 559 | 6 |

### Materials and methods

### In vitro Protein Kinase Assay

The effect of the thiazole derivatives was tested on recombinant, human protein kinases in *in vitro* protein kinase assays. The protein kinases were expressed in Sf9 insect cells as human recombinant GST-fusion proteins or as His-tagged proteins by means of the baculovirus expression system.

The kinase activity was measured with a radioisotopic protein kinase assay by measuring the incorporation of ³³P-phosphate into proteins or peptide substrates. The assays were performed in FlashPlate^{™} microtiter scintillation plates and incorporation of ³³P-phosphate was determined with a microplate scintillation counter.

### Cellular Receptor Tyrosine Kinase Assay

The effect of thiazole derivatives was tested by determining the inhibition of different receptor tyrosine kinases (RTKs) in various cell lines which expressed the following growth factor receptors: EGF-R, PDGF-R, TIE2, IGF-1R, EPHB4, and VEGF-R2. Receptor autophosphorylation was induced by specific ligands for each receptor. Stimulation of cells resulted in maximal autophosphorylation in control cells (high control) without inhibitor. Test compounds were applied to cells prior to stimulation. Cells were lysed using a standard lysis buffer preserving the distinct phosphoprotein levels. RTK-phosphorylation was quantified via sandwich ELISA using receptor-specific capture antibodies and a phosphotyrosine antibody.

Sigmoidal inhibitor curves based on relative inhibition compared with phosphorylation levels under high control conditions were generated which allowed the determination of IC₅₀ values for each test compound.

### Cellular Aurora-B Kinase Assay

The effect of thiazole derivatives was tested in a cellular Aurora-B assay by measuring the effect of the test compounds on the endoreduplication (EndoR) of genomic DNA. Endoreduplication is detectable in cells as DNA-content higher then 4 n. Propidium Iodine (PI) was used to quantify the DNA content using a fluorescence activated cell sorter (FACS).

In the experiment, HT29 colon-carcinoma cells were treated with test compounds at different concentrations for 3 days. On day 5 cells were harvested and fixed in methanol. On day 6 cells were rehydrated and incubated with RNAse A and PI. Incorporated PI was detected by FACS measuring fluorescence emission at 650 nm upon excitation at 488 nm. For each compound concentration the percentage of EndoR-population as compared to the whole cell population was determined. For estimation of IC₅₀ values of Aurora-B inhibition the percentages of EndoR-populations were plotted versus compound concentrations.

### Cellular Aurora-B Kinase Histone H3 Phosphorylation Assay

The effect of compounds was tested in a cellular Aurora-B assay measuring phosphorylation of the Aurora B-substrate protein Histone H3 at Serine 10 (HisH3-pS10). Inhibition of Aurora B results in reduction of HisH3-pS10 which was detected in a specific immuno-assay.

In the experiment, HT-29 colon-carcinoma cells were seeded on day 1 and on day 2 test compounds at different concentrations were added. Cells were incubated with test compounds for 1 hour. Subsequently, Calyculin A was added for 30 min. For DELFIA®-detection (PerkinElmer) of HisH3-pS10, lysates were transferred to a microtiterplate and incubated with detecting antibody directed against HisH3-pS10 and Europium-labelled secondary anti-IgG-antibody. Emission at 615 nm was measured upon excitation at 340 nm and the percentage of inhibition was calculated for each concentration of the test compounds relative to controls without inhibitor. Mean values of HisH3-pS10 percentage were plotted versus compound concentration for calculation of IC₅₀-values.

### Results

In vitro Protein Kinase Assay

The compounds of the present invention show IC₅₀ values lower than 500 nM on at least one kinase selected from Aurora-A, Aurora-B, EGF-R, ERBB2, PDGFR, FLT3, IGF1-R, VEGF-R2, VEGF-R3, EPHB4, TIE2, FAK, and SRC or display a beneficial activity profile by inhibiting at least two kinases from at least two different molecular mechanisms of tumor progression with IC₅₀ values lower than 500 nM.

The compounds of the present invention show IC₅₀ values lower than 10 µM in the Cellular Receptor Tyrosine Kinase Assay and / or the Cellular Aurora-B Kinase Assay.

## Claims

1. A compound of the general formula (I) and salts and physiologically functional derivatives thereof, 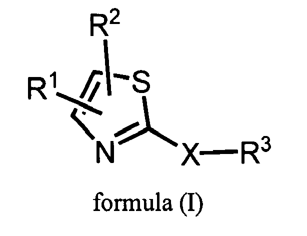 wherein
R² is attached at the 4- or 5-position of the thiazole ring and is hydrogen, alkyl, halogen, cyano, alkoxy, haloalkyloxy, or alkylamino;
R¹ is attached at the 4- or 5-position of the thiazole ring and independently represents one of the following groups of the general formula (II): 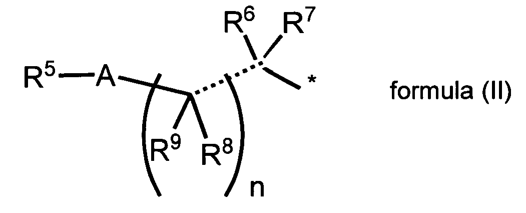 wherein
the dotted line represents a single or double bond;
* indicates the point of attachment to the thiazole ring;
n is 1, 2, or 3;
A independently represents a divalent linkage group selected from ←C(=O)-, ←C(=S)-, ←S(=O)-, ←S(=O)₂-, ←C(=O)O-, ←C(=O)NR¹²-, ←NR¹²C(=O)-, ←NR¹²C(=O)NR¹³-; ←NR¹²C(=O)O-, ←NR¹²NR¹³C(=O)-, ←NR¹²OC(=O)-, or ←ONR¹²C(=O)-, ←NR¹²S(=O)₂-, and where ← indicates the point of attachment to R⁵;
R⁵ is hydrogen, alkyl, cycloalkyl, cycloalkylalkyl, aryl, arylalkyl, heteroaryl, heteroarylalkyl;
or A and R⁵ together form an isoindol-1,3-dione-2-yl-ring which may be independently substituted by one to three substituents selected from halogen, CF₃, CHF₂, CH₂F, OCF₃, cyano, hydroxy, amino, nitro, alkoxy, alkylamino, alkyl, ethynyl, alkoxy, haloalkyloxy;
R⁶ is hydrogen, halogen, cyano, hydroxy, amino, alkyl, alkoxy, alkylamino, cycloalkyl, haloalkyloxy, or haloalkyl;
R⁷ is hydrogen, halogen, cyano, hydroxy, amino, alkyl, alkoxy, alkylamino, cycloalkyl, haloalkyloxy, or haloalkyl, or R⁷ is absent in case the dotted line represents a double bond;
R⁸ is hydrogen, halogen, cyano, hydroxy, amino, alkyl, alkoxy, alkylamino, cycloalkyl, haloalkyloxy, or haloalkyl,
or R⁸ and R⁶ together form a 3- to 8-membered saturated or unsaturated monocyclic ring, which may contain further heteroatoms selected from N, O or S, wherein one or more carbon atoms may be independently substituted by one to three substituents selected from halogen, CF₃, CHF₂, CH₂F, OCF₃, cyano, hydroxy, amino, nitro, alkoxy, alkylamino, alkyl, ethynyl, alkoxy, or haloalkyloxy;
R⁹ is hydrogen, halogen, cyano, hydroxy, amino, alkyl, alkoxy, alkylamino, cycloalkyl, haloalkyloxy, or haloalkyl, or R⁹ is absent in case the dotted line represents a double bond;
R¹² is hydrogen, alkyl, cycloalkyl, aryl, arylalkyl, or heteroaryl;
R¹³ is hydrogen; alkyl, or cycloalkyl;
X independently represents a divalent linkage group selected from S, O, NR⁴, SO, or SO₂;
R⁴ is hydrogen, alkyl, cycloalkyl, cycloalkylalkyl, or heterocyclyl;
R³ independently represents one of the following groups, which may be independently substituted by one to three substituents R¹⁸ via the aromatic carbon atoms: 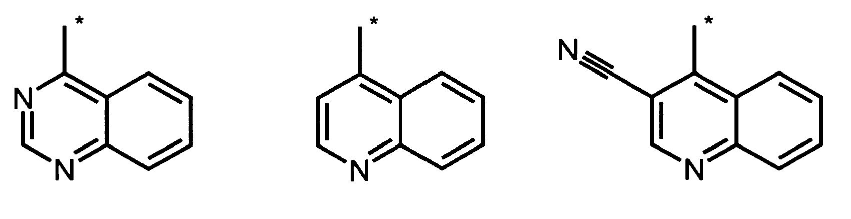 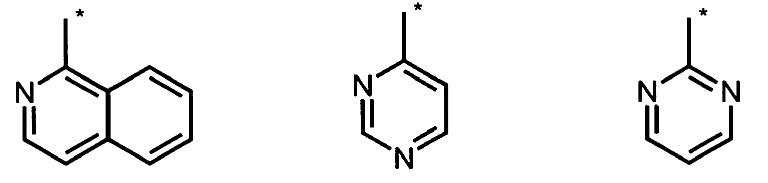 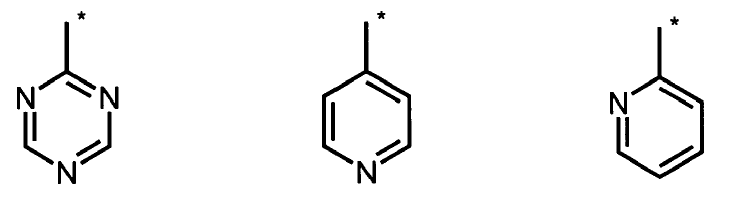 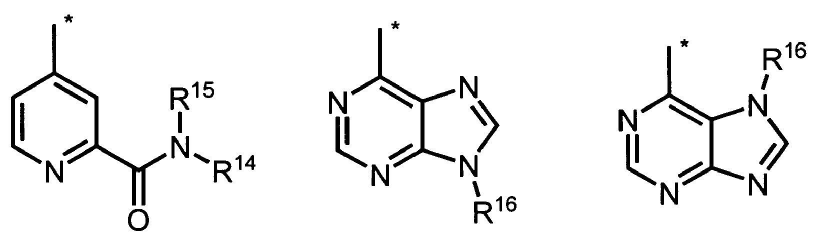 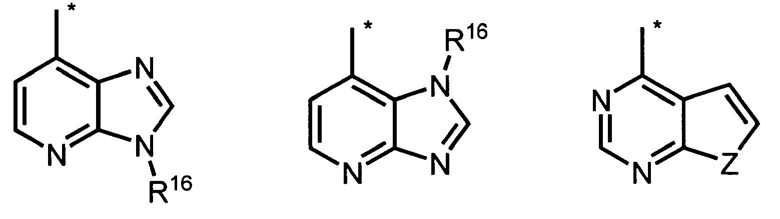 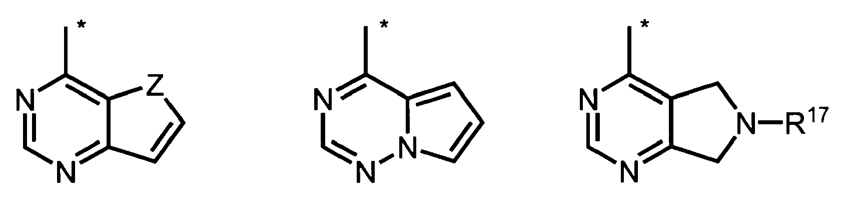 wherein
* indicates the point of attachment to X;
Z is O, NR¹⁶, or S;
R¹⁴ is hydrogen, alkyl, cycloalkyl, heterocyclyl, or -E¹-R¹⁹;
R¹⁵ is hydrogen or alkyl;
R¹⁶ is hydrogen, alkyl, cycloalkyl, heterocyclyl, aryl, or heteroary;
R¹⁷ is hydrogen or -E²-R¹⁹;
E¹ is absent or represents a divalent linkage group selected from -0-, -N(R¹⁵)-, ←C(=O)-, ←C(=S)-, ←S(=O)-, ←S(=O)₂-, ←C(=O)O-, ←C(=O)NR¹⁶-, ←NR¹⁶C(=O)-, ←NR¹⁶C(=O)NR⁴-; ←NR¹⁶C(=O)O-, ←NR¹⁶S(=O)₂-, where ← indicates the point of attachment to the nitrogen atom in the pyridine-2-carboxylic acid amide;
E² is absent or represents a divalent linkage group selected from -O-, -N(R¹⁵)-, ←C(=O)-, ←C(=S)-, ←S(=O)-, ←S(=O)₂-, ←C(=O)O-, ←C(=O)NR¹⁶-, ←NR¹⁶C(=O)-, ←NR¹⁶C(=O)NR⁴-; ←NR¹⁶C(=O)O-, ←NR¹⁶S(=O)₂-, where ← indicates the point of attachment to the nitrogen atom of 6,7-dihydro-5H-pyrrolo[3,4-d]pyrimidine;
R¹⁸ is hydrogen, halogen, hydroxy, alkoxy, haloalkyloxy, alkylamino, alkyl, cycloalkyl, haloalkyl, cyano, nitro, or -E³-R¹⁹_{;}
E³ is absent or represents a divalent linkage group selected from -O-, -N(R¹⁵)-, ←C(=O)-, ←C(=S)-, ←S(=O)-, ←S(=O)₂-, ←C(=O)O-, ←C(=O)NR¹⁶-, ←NR¹⁶C(=O)-, ←NR¹⁶C(=O)NR⁴-; ←NR¹⁶C(=O)O-, ←NR¹⁶S(=O)₂-, where ← indicates the point of attachment to an aromatic carbon atom of the R³ residue;
R¹⁹ is H or represents a group selected from the general formula (III) 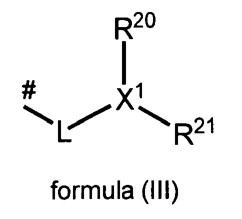 wherein
# indicates the point of attachment to E¹, or E² or E³;
L is absent or represents a divalent linkage group selected from alkylene, cycloalkylene, heterocyclylene, arylene, or heteroarylene, wherein one or more of the (-CH₂-) groups may be replaced by an oxygen or a NR¹⁵, and wherein one or more carbon atoms may be independently substituted by one or two substituents selected from halogen, hydroxy, alkoxy, haloalkyloxy, phoshonooxy, phoshonooxyalkyl;
X¹ is CH, N, or O;
R²⁰ is hydrogen, alkyl, cycloalkyl, heterocyclyl, aryl, heteroaryl, or X¹, R²⁰ and R²¹ together form a 3- to 10-membered monocyclic or bicyclic saturated or unsaturated ring, which may contain further heteroatoms selected from N, O or S, wherein one or more carbon atoms may be independently substituted by R²² and each of the nitrogen atoms may be independently substituted by R²³;
R²¹ is H, alkyl, cycloalkyl, heterocyclyl, aryl, or heteroaryl;
R²² is halogen, alkoxy, alkyl, cycloalkyl, haloalkyl, haloalkyloxy, phosphonooxy, or phosphonooxyalkyl;
R²³ is hydrogen, alkyl, -CO-CH₂-OH, or -CO-CH₂-O-PO(OH)₂;
an alkyl group, if not stated otherwise, denotes a linear or branched C₁-C₆-alkyl, a linear or branched C₂-C₆-alkenyl or a linear or branched C₂-C₆-alkynyl group, which may be substituted by one or more substituents R';
the C₁-C₆-alkyl, C₂-C₆-alkenyl and C₂-C₆-alkynyl residues may be selected from the group comprising -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R')₃, -C₂(R')₅, -CH₂-C(R')₃, -C₃(R')₇, -C₂H₄-C(R')₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C=CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C=CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;
R' independently represents H, -CO₂R", -CONHR", -CR"O, -SO₂NR", -NR"-CO-haloalkyl, -NO₂, -NR"-SO₂-haloalkyl, -NR"-SO₂-alkyl, -SO₂-alkyl, -NR"-CO-alkyl, -CN, alkyl, cycloalkyl, aminoalkyl, alkylamino, alkoxy, -OH, -SH, alkylthio, hydroxyalkyl, hydroxyalkylamino, halogen, haloalkyl, haloalkyloxy, aryl, arylalkyl or heteroaryl;
R" independently represents H, haloalkyl, hydroxyalkyl, alkyl, cycloalkyl, aryl, heteroaryl or aminoalkyl;
an alkylene group denotes a divalent linear or branched C₁-C₆-alkylene, a linear or branched C₂-C₆-alkenylene or a linear or branched C₂-C₆-alkynylene group, which may be substituted by one or more substituents R';
a cycloalkylene group denotes a divalent non-aromatic ring system containing three to eight carbon atoms, wherein one or more of the carbon atoms in the ring may be substituted by a group E, E being O, S, SO, SO₂, N, or NR", R" being as defined above;
a heterocyclylene group denotes a 3 to 8-membered divalent heterocyclic nonaromatic group which contains at least one heteroatom selected from O, N, and S, wherein the heterocyclylene group may be fused to another non-aromatic ring and may be substituted by one or more substituents R', wherein R' is as defined above;
an arylene group denotes an aromatic divalent group having five to fifteen carbon atoms, which may be substituted by one or more substituents R', wherein the arylene group may be fused to another aromatic ring, and R' is as defined above;
a heteroarylene group denotes a divalent 5- or 6-membered heterocyclic group which contains at least one heteroatom selected from O, N, and S, wherein the heterocyclylene group may be fused to another aromatic ring and may be substituted by one or more substituents R', wherein R' is as defined above;
a cycloalkyl group denotes a non-aromatic ring system containing three to eight carbon atoms, wherein one or more of the carbon atoms in the ring may be substituted by a group E, E being O, S, SO, SO₂, N, or NR", R" being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅, morpholine-4-yl, piperazinyl, 1-alkylpiperazine-4-yl;
an alkoxy group denotes an O-alkyl group, the alkyl group being as defined above;
a alkylthio group denotes a S-alkyl group, the alkyl group being as defined above;
a haloalkyl group denotes a alkyl group which is substituted by one to five halogen atoms, the alkyl group being as defined above;
a hydroxyalkyl group denotes a HO-alkyl group, the alkyl group being as defined above;
a haloalkyloxy group denotes an alkoxy group which is substituted by one to five halogen atoms, the alkyl group being as defined above;
a hydroxyalkylamino group denotes a (HO-alkyl)₂-N- group or HO-alkyl-NH-group, the alkyl group being as defined above;
an alkylamino group denotes a HN-alkyl or N-dialkyl group, the alkyl group being as defined above;
a halogen group is fluorine, chlorine, bromine, or iodine;
an aryl group denotes an aromatic group having five to fifteen carbon atoms, which may be substituted by one or more substituents R' and may be fused to another aromatic ring, wherein R' is, as defined above;
a heteroaryl group denotes a 5- or 6-membered heterocyclic group, which contains at least one heteroatom selected from O, N, and S , which may be fused to another aromatic ring, and which may be substituted by one or more substituents R', wherein R' is as defined above;
a heterocyclyl group denotes a 3 to 8-membered heterocyclic non-aromatic group, which contains at least one heteroatom selected from O, N, and S, which may be fused to another non-aromatic ring, and which may be substituted by one or more substituents R', wherein R' is as defined above;
a phosphonooxy group is -O-P(=O)(OH)₂ or a salt thereof;
a phosphonooxyalkyl group denotes an -alkyl-O-P(=O)(OH)₂ group or a salt
thereof, alkyl being as defined above.

2. The compound according to claim 1, wherein X is NH.

3. The compound according to claim 2, wherein R¹ is attached at the 5-position of the thiazole ring.

4. The compound according to claim 2, wherein R¹ is attached at the 4-position of the thiazole ring.

5. A compound according to any one of claims 1 to 4 for the use as a medicament.

6. A composition containing a compound according to any one of claims 1 to 4 and a pharmaceutically acceptable carrier or diluent.

7. The use of a compound according to any one of claims 1 to 4, or a composition of claim 6 for the preparation of a medicament for the treatment or prevention of cancer.

8. The use of a compound according to any of claims 1 to 4, or a composition of claim 6 for the preparation of a medicament for treating, relieving, and/or preventing solid tumors in particular breast, bladder, colorectal, lung, prostate, pancreatic and renal cancer, or leukemias or lymphomas.

9. The use of a compound according to any one of claims 1 to 4 or a composition of claim 6 for the preparation of a medicament for the treatment of diseases which are cured or relieved by the inhibition of one or several kinases.

10. The use of a compound according to any one of claims 1 to 4 or a composition of claim 6 for the preparation of a medicament for the treatment of diseases which are cured or relieved by the inhibition of one ore several kinases like: Aurora-A, Aurora-B, EGF-R, ERBB2, PDGFR, FLT3, IGF1-R, VEGF-R2, VEGF-R3, EPHB4, TIE2, FAK, and SRC.
